# EUROPÄISCHE PATENTSCHRIFT

(11) **EP 2 037 904 B1**
(45) Veröffentlichungstag und Bekanntmachung des Hinweises auf die Patenterteilung: **19.09.2012**
(21) Anmeldenummer: 07764424.3
(22) Anmeldetag: 03.07.2007
(51) Int. Cl.: A61K 31/135, A61P 43/00

(54) **BASISCHE ACETOPHENONE ALS HEMMSTOFFE VON NO-SYNTHASEN**
BASIC ACETOPHENONES AS INHIBITORS OF NO-SYNTHASES
ACÉTOPHÉNONES BASIQUES EN TANT QU'INHIBITEURS DE NO-SYNTHASES

(30) Priorität: 07.07.2006 DE 102006031813
(43) Veröffentlichungstag der Anmeldung: 25.03.2009
(73) Patentinhaber: Christian-Albrechts-Universität zu Kiel, 24118 Kiel (DE)
(72) Erfinder: CLEMENT, Bernd, 24106 Kiel (DE); HEBER, Dieter, 24113 Molfsee (DE); BLUHM, Ullvi, 24119 Kronshagen (DE); BUSS, Uwe, 48167 Münster (DE); FRIEDRICH, Friederike, 24114 Kiel (DE)
(74) Vertreter: Müller Fottner Steinecke
(86) Internationale Anmeldenummer: PCT/DE2007/001176
(87) Internationale Veröffentlichungsnummer: WO 2008/003299

(56) Entgegenhaltungen:
- EP-A- 0 885 869
- WO-A-2004/078755
- AU-B2- 618 439
- DE-A1- 2 112 716
- GB-A- 1 475 314

## Beschreibung

Die Erfindung betrifft basische Acetophenone als Hemmstoffe von NO-Syntasen und deren Verwendung zur Herstellung von Arzneimitteln.

NO-Synthasen (NOS) katalysieren die Umsetzung der Aminosäure L-Arginin zu Stickstoffmonoxid (NO) und L-Citrullin. Aufgrund der Beteiligung einer Überproduktion von Stickstoffmonoxid in zahlreichen pathophysiologischen Prozessen (Stuehr, 1999, Biochim Biophys Acta, 1411, 217-230) wird die Entwicklung von Hemmstoffen der NO-Synthasen als viel versprechende Strategie angesehen (Babu und Griffith, 1998, Curr Opin Chem Biol, 2, 491-500). Eine verstärkende Wirkung auf das Krankheitsgeschehen von übermäßig produziertem Stickstoffmonoxid besteht beispielsweise bei der Alzheimerkrankheit (Dorheim et al., 1994, Biochem Biophys Res Commun, 205, 659-665), Diabetes mellitus (Li und Förstermann, 2000, J Pathol, 190, 244-254), der Parkinsonkrankheit (Schulz et al., 1995, J Neurochem, 64, 936-939), multipler Sklerose (Heales et al., 1999, Biochim Biophys Acta, 1410, 215-228), rheumatoider Arthritis (Stefanovic-Racic et al., 1993, Arthritis Rheum, 36, 1036-1046), dem septischen Schock (Crossin, 1991, Trends Biochem Sci, 1991, 16, 81-82), Asthma bronchiale (Ligget et al., 1995, Am J Respir Crit Care Med, 152, 394-402), chronisch-entzündlichen Darmerkrankungen (Boughton-Smith et al., 1993, Lancet, 342, 338-340), und der Migräne (Olesen et al., 1994, Trends Pharmacol Sci, 15, 149-153). Vor diesem Hintergrund wurden in den letzten Jahren zahlreiche Hemmstoffe entwickelt, die sich z. T. auch in Tiermodellen für die erwähnten Krankheiten als wirksam erwiesen haben. Besonders viel versprechend ist die pharmakologische Blockade von NO-Synthasen bei solchen Schmerzgeschehen, die eine Entzündungskomponente beinhalten. Hier wirken im Tiermodell NOS-Inhibitoren sowohl auf der Nociceptorebene als auch auf der Weiterleitungsebene (Stegmann et al., 2001, Anasthesiol Intensivmed Notfallmed Schmerzther, 36, 276-281). Leider konnte bislang keiner dieser Hemmstoffe zum Arzneistoff weiterentwickelt werden (Roman et al., 2002, Chem Rev, 102, 1179-1189).

Aufgabe der vorliegenden Erfindung ist es daher, Verbindungen bereitzustellen, die insbesondere als Hemmstoffe für NO-Synthasen wirken und sich als pharmazeutische Wirkstoffe in Arzneimitteln eignen.

Erfindungsgemäß wird diese Aufgabe gelöst durch die die Merkmale des Anspruchs 1. Die Unteransprüche geben vorteilhafte Ausgestaltungen der Erfindung an.

Die erfindungsgemäße Verwendung der beanspruchten Substanzen betrifft auch diejenigen Substanzen, die als Tautomere in Form ihres Salzes oder ihrer Base vorliegen können

Als ein charakteristisches Beispiel für eine der erfindungsgemäßen Substanzen, die inhibierend auf die NO-Synthase wirkt, ist 4'-(4-Bromphenyl)-3-dimethylaminopropiophenonhydrochlorid zu nennen:

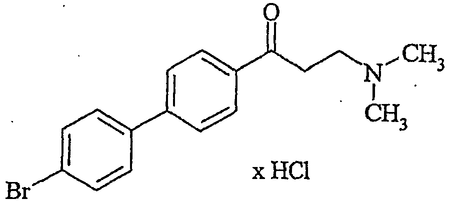

In einem *in vitro* Testsystem, bei dem L-Arginin in Anwesenheit von NO-Synthase zu L-Citrullin und Stickstoffmonoxid umgesetzt wird (Griess-Assay), bewirkt 4'-(4-Bromphenyl)-3-dimethylaminopropiophenonhydrochlorid eine konzentrationsabhängige Hemmung der im Zentralnervensystem vorkommenden bNOS von bis zu 88 %. Die Verbindung ist zwar literaturbekannt (Niwa, H., 1957, Tohoku Yakka Daigaku Kiyo, 4, 69-78), eine NOS-inhibierende Wirkung ist bisher jedoch nicht beschrieben worden.

Als ein weiteres Beispiel für eine der erfindungsgemäßen Substanzen ist die Verbindung 4'-(4-Bromphenyl)-4-dimethylaminobutyrophenonhydrochlorid zu nennen. Diese Substanz ist nicht literaturbekannt und bewirkt eine ebenfalls konzentrationsabhängige Hemmung der bNOS von bis zu 99 %.

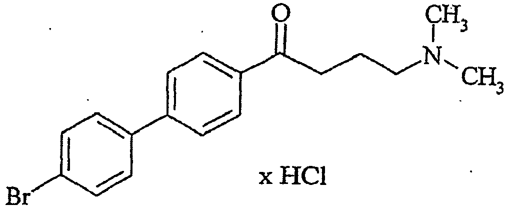

Die erfindungsgemäßen basischen Acetophenone stellen somit hochwirksame niedermolekulare Hemmstoffe von NO-Synthasen dar. Sie sind leicht und kostengünstig über Standardmethoden und in hoher Reinheit zu synthetisieren. Überraschenderweise hat sich herausgestellt, dass die erfindungsgemäßen Substanzen eine Selektivität für die im Zentralnervensystem vorkommende bNOS aufweisen, die mit neuronalen Krankheiten wie Alzheimerkrankheit, Parkinsonkrankheit, multipler Sklerose und Migräne in Verbindung gebracht wird. In diesem Zusammenhang ist es von besonderer Bedeutung, dass die erfindungsgemäßen Hemmstoffe, aliphatische Amine mit lipophilem Rest, nach oraler Aufnahme und Resorption über das Darmepithel dazu in der Lage sind, die Blut-Hirn-Schranke zu überwinden.

Die konzentrationsabhängige Wirksamkeit der erfindungsgemäßen Substanzen wird in den vorliegenden Abbildungen genauer dargestellt. Es zeigt dabei
- Fig. 1: die Aktivität der bNOS in Abhängigkeit von der Konzentration an 4'-(4-Bromphenyl)-3-dimethylaminopropiophenonhydrochlorid
- Fig. 2: die Aktivität der bNOS in Abhängigkeit von der Konzentration an 4'-(4-Bromphenyl)-4-dimethylaminobutyrophenonhydrochlorid

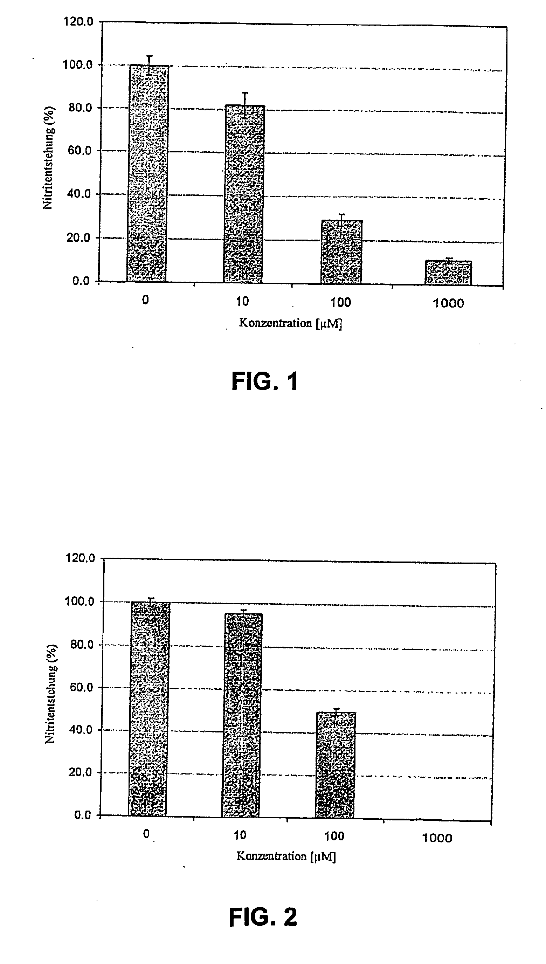

Sowohl in Fig. 1 als auch in Fig. 2 wird die Aktivität der bNOS anhand der Nitritentstehung gemessen. Nitrit ist das Endprodukt von Stickstoffmonoxid in sauerstoffhaltiger wässriger Lösung. Kontrollinkubationen mit sämtlichen Cofaktoren, jedoch ohne Hemmstoffzusatz, wurden auf 100 % festgelegt. Die Werte sind als Mittelwerte +/- Standardabweichung von drei Inkubationen angegeben, die jeweils doppelt vermessen wurden.

Die Synthese der erfindungsgemäßen NO-Syntase-Hemmstoffe und die Durchführung der z.T. in Fig. 1 und Fig. 2 dargestellten *in vitro* Tests zu deren Wirksamkeit werden im Folgenden näher erläutert.

Sämtliche erfindungsgemäßen Substanzen können nach gängigen Methoden hergestellt werden. Die Verbindungen mit der Kettenlänge n = 2 können durch Umsetzung teils substituierter Acetophenone mit Dimethylammoniumchlorid und Paraformaldehyd im Sinne einer Mannich-Reaktion erhalten werden. Die Acetophenonkomponenten wurden, soweit nicht kommerziell erhältlich, durch Friedel-Crafts-Acylierungen gewonnen. Die Reaktion ist im Folgenden beispielhaft für die Verbindung 4'-(4-Bromphenyl)-3-dimethylaminopropiophenonhydrochlorid dargestellt:

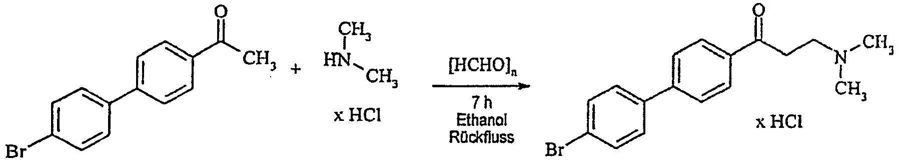

Verbindungen mit der Kettlänge n = 3 wurden durch Reaktion von substituierten 4-Chlorbutyrophenonderivaten mit Dimethylamin im Sinne einer nucleophilen Substitution erhalten. Diese Reaktion ist im Folgenden beispielhaft für die Verbindung 4'-(4-Bromphenyl)-4-dimethylaminobutyrophenonhydrochlorid angegeben:

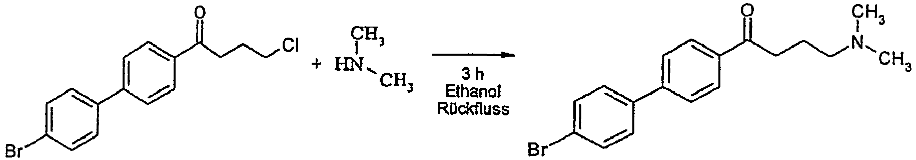

Die angegebene freie Base wurde anschließend als Hydrochlorid gefällt. Die Butyrophenonkomponenten wurden, soweit nicht kommerziell erhältlich, ebenfalls durch Friedel-Crafts-Acylierungen gewonnen.

Die Schmelzpunkte der synthetisierten Substanzen wurden an Schmelzpunktapparatur Büchi 510 Gerät mit Mikroheiztisch Thermovar (Firma Reichert) aufgenommen. NMR-Spektren wurden an einem Kernresonanzspektrometer Broker ARX 300 aufgenommen. Die Aufnahmen von IR-Spektren (als KBr-Presslinge) erfolgten an einem Perkin-Elmer FT-IR 16 PC Spectrometer. Massenspektren wurden an einem Gerät vom Typ Hewlett-Packard 5989 aufgenommen. Elementaranalysen wurden am Institut für Anorganische Chemie der CAU zu Kiel mittels eines CHNS-Analysators der Firma Hekatech GmbH bestimmt. Soweit nicht anders angegeben wurden die Chemikalien in höchster Reinheit bei der Firma Sigma-Aldrich GmbH bezogen. (6*R*)-5,6,7,8-Tetrahydro-L-biopterin sowie die NO-Synthasen (bNOS, iNOS, eNOS) wurden bezogen von der Firma Axxora GmbH. Von der Firma Merck KGaA stammten Calciumchlorid Hexahydrat, Dimethylsulfoxid, Ethylendiamintetraessigsäure, Naphthylethylendiamindihydrochlorid sowie Nicotinamidadenindinucleotid-Tetranatriumsalz (reduzierte Form). Magnesiumchlorid Hexahydrat sowie L-Arginin wurde bei der Firma Fluka Chemie GmbH bezogen. Acetonitril stammte von der Firma LGC Promochem GmbH. Calmodulin (gereinigt, aus Schweinehirn) stammte von der Firma Roche Diagnostics GmbH. Die Vermessungen erfolgten an einem Spektralphotometer Varian Cary 50 Bio mit Water Peltier System PCB 150 und Einmalküvetten mikro, Zentrumshöhe 15 mm, Volumen 70-550 µl (Firma Varian GmbH).

Die Inkubationen der synthetisierten Substanzen mit den jeweiligen NO-Synthasen wurden wie folgt durchgeführt:
die Standard-Inkubationsansätze hatten folgende Zusammensetzung:
   - 60 µl einer Lösung aus dem Enzym (es handelte sich stets um rekombinante humane I-soenzyme), dem Substrat sowie den Cofaktoren
   - 10 µl des jeweiligen Inkubationspuffers
   - 10 µl einer zuvor in achtfacher Konzentration hergestellten Lösung der potenziellen Hemmstoffe, wobei ein Anlösen unter Verwendung von 10 % Dimethylsulfoxid erfolgte;
bei Kontrollansätzen wurden statt der Hemmstoff-Lösung weitere 10 µl Puffer pipettiert.

Die Ansätze wurden nach Herstellung für 20 min (iNOS und eNOS) bzw. für 30 min (bNOS) im Schüttelwasserbad bei 37 °C inkubiert. Anschließend wurden jeweils 20 µl einer Lösung zupipettiert, die Natriumpyruvat (1,6 mM, im jeweiligen Inkubationspuffer) und Lactatdehydrogenase (20 U/ml, im jeweiligen Inkubationspuffer) enthielt. Nach einer weiteren Inkubation für 20 min im Schüttelwasserbad bei 37 °C wurden die Inkubationen durch Zugabe von je 50 µl eiskalten Acetonitrils abgestoppt. Dann wurden etwaige Niederschläge abzentrifugiert (ungekühlte Laborzentrifuge, 10.000 rpm, 5 min). Jeweils 120 µl der Überstände wurden mit 24 µl einer Lösung aus Sulfanilamid und Naphthylethylendiamindihydrochlorid versetzt. Nach 5 min Inkubation bei Raumtemperatur wurden die Lösungen bei der Wellenlänge 543 nm vermessen, wobei als Blindwert die erwähnte nicht-enzymhaltige Probe verwendet wurde. Beim Griess-Assay wird ausgenutzt, dass Stickstoffmonoxid sich in wässrigen Lösungen äquimolar zu Nitrit umsetzt (Ignarro et al., 1993, Proc Natl Acad Sei USA, 90, 8103-8107). Das Nitrit kann quantitativ durch Reaktion mit Sulfanilamid und Naphthylethylendiamin in saurem Milieu bestimmt werden. Es entsteht ein Azofarbstoff, der spektralphotometrisch vermessen werden kann. Die Lösung enthielt Naphthylethylendiamindihydrochlorid 5,8 mM und Sulfanilamid 52 mM. Als Lösungsmittel wurde 1N HCl verwendet. Unter Bezugnahme auf die Kontroll-Ansätze, die keine Hemmstoffe enthielten, wurden die relativen Nitritbildungen ermittelt. Eine Übersicht über sämtliche in den Standard-Inkubationsansätzen verwendeten Zusätze und Konzentrationen von Substrat und Cofaktoren liefert die unten stehende Tabelle, als Lösungsmittel wurden die jeweiligen Inkubationspuffer verwendet. Im Falle des Tetrahydrobiopterins wurde aus Stabilitätsgründen 0,1 mM HCI benutzt:

| **Inkubationszusatz** | **Endkonzentration** | **Lagerung** |
|---|---|---|
| L-Arginin (bNOS) | 0,5 mM | 4°C |
| L-Arginin (iNOS und eNOS) | 1mM | 4°C |
| Tetrahydrobiopterin | 10 µM | -20 °C |
| CaCl₂ (bNOS und eNOS) | 0,5 mM | 4°C |
| Calmodulin (bNOS und eNOS) | 10 µg/ml | -20 °C |
| Flavinadenindinucleotid, reduzierte Form | 5 µM | -20 °C |
| Flavinmononucleotid, reduzierte Form | 5 µM | -20 °C |
| MgCl₂ (iNOS) | 1 mM | 4°C |
| Nicotinamiddinucleotid, reduzierte Form | 0,5 mM | -20 °C |

### Puffer A (Inkubationen mit bNOS):

Der für bNOS-Inkubationen verwendete Puffer hatte folgende Zusammensetzung: Triethanolamin 50 mM, 2-Mercaptoethanol 10 mM, 3-[(3-Cholamidopropyl)dimethylammonio]-propansulfat 1 mM, Ethylendiamintetraessigsäure 0,5 mM. Nach Herstellung wurde der pH-Wert auf 7,0 eingestellt.

### Puffer B (Inkubationen mit iNOS und eNOS):

Puffer B bestand aus einer 50 mM und auf pH = 7,5 eingestellten Triethanolamin-Lösung.

## Patentansprüche

1. Verwendung eines Stoffs mit der allgemeinen Strukturformel 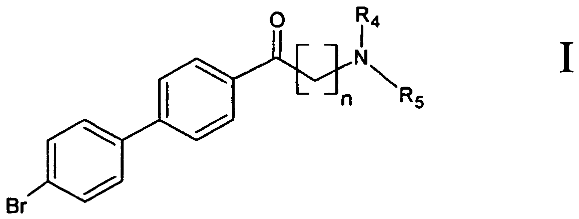 oder eines Stoffs mit der allgemeinen Strukturformel 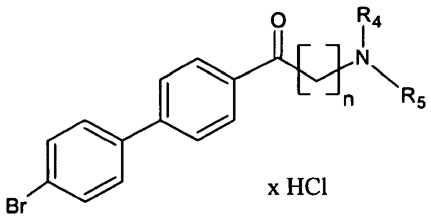 zur Herstellung eines Arzneimittels zur Behandlung von Alzheimer, Parkinson, septischem Schock, Asthma bronchiale oder Migräne,
wobei
- R₄ und R₅ jeweils eine Methylgruppe und
- n gleich 2 oder 3 ist.

2. Verwendung nach Anspruch 1, **dadurch gekennzeichnet, dass** der Stoff 4'-(4-Bromphenyl)-3-dimethylaminopropiophenonhydrochlorid mit der Strukturformel (II) 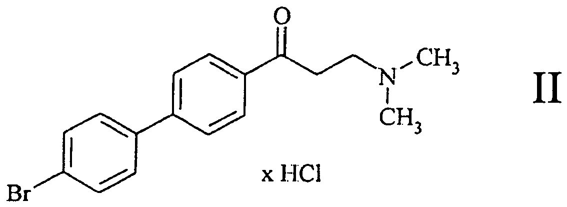 ist.

3. Verwendung nach Anspruch 1, **dadurch gekennzeichnet, dass** der Stoff 4'-(4-Bromphenyl)-4-dimethylaminobutyrophenonhydrochlorid mit der Strukturformel (III) 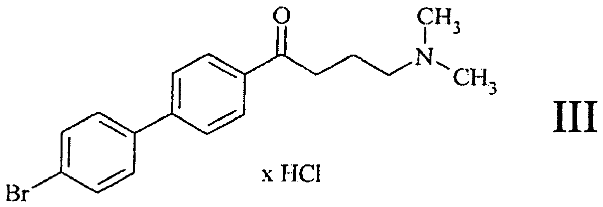 ist.

4. Verwendung nach einem der vorhergehenden Ansprüche, **dadurch gekennzeichnet, dass** der Stoff ein Tautomer des Stoffs mit der Strukturformel (II) oder (III) ist.

## Claims

1. Use of a substance with the general structural formula 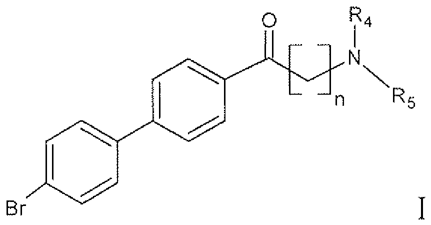 or a substance with the general structural formula 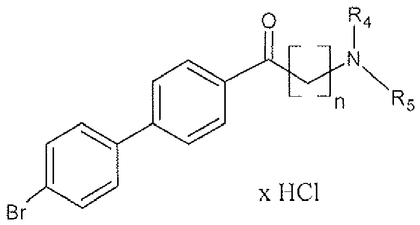 for the manufacture of a medicament for the treatment of Alzheimer's disease, Parkinson's disease, septic shock, bronchial asthma or migraine,
wherein
- R₄ and R₅ are each a methyl group and
- n is equal 2 or 3.

2. The use of claim 1, **characterized in that** the substance is 4'-(4-Brominephenyl)-3-dimethylaminopropiophenonehydrochloride with the structural formula (II)

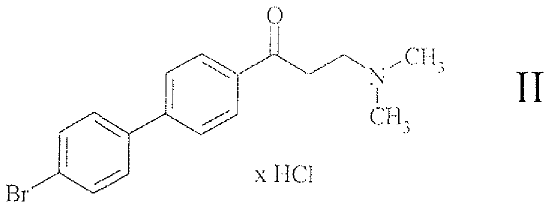

3. The use of claim 1, **characterized in that** the substance is 4'-(4-Brominephenyl)-4-dimethylaminobutyrophenonehydrochloride with the structural formula (III).

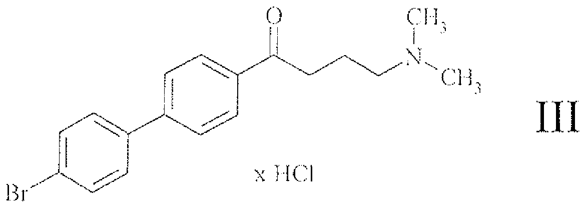

4. The use of any one of the preceding claims, **characterized in that** the substance is a tautomer of the substance with the structural formula (II) or (III).

## Revendications

1. Utilisation d'une substance de formule développée générale 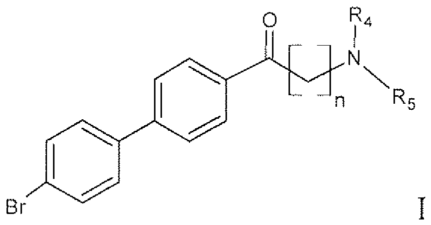 ou d'une substance de formule développée générale 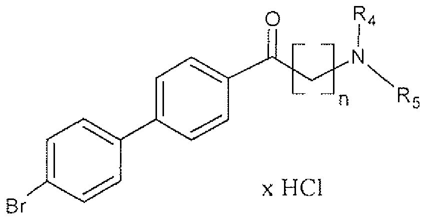 pour la fabrication d'un médicament pour le traitement de la maladie d'Alzheimer, de la maladie de Parkinson, du choc septique, de l'asthme bronchique ou de la migraine, dans laquelle
- R₄ et R₅ sont chacun un groupe méthyle, et
- n est égal à 2 ou 3.

2. Utilisation selon la revendication 1, **caractérisée en ce que** la substance est le chlorhydrate de 4'-(4-bromophényl)-3-diméthylaminopropiophénone, de formule développée (II)

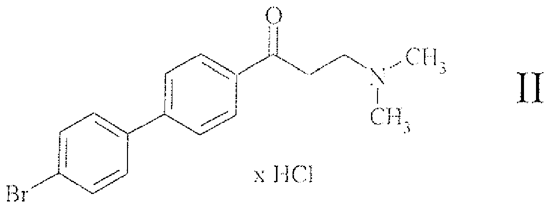

3. Utilisation selon la revendication 1, **caractérisée en ce que** la substance est le chlorhydrate de 4'-(4-bromophényl)-4-diméthylaminobutyrophénone, de formule développée (III)

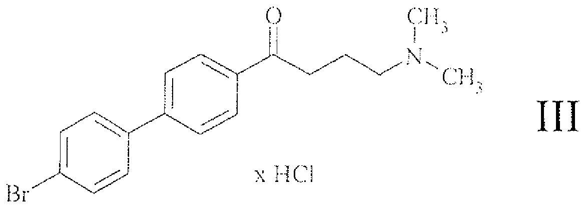

4. Utilisation selon l'une quelconque des revendications précédentes, **caractérisée en ce que** la substance est un tautomère de la substance selon la formule développée (II) ou (III).
